(19) Europäisches Patentamt
European Patent Office
Office européen des brevets

(11) Publication number: 0 240 274
A2

(12) # EUROPEAN PATENT APPLICATION

(21) Application number: 87302711.4

(22) Date of filing: 30.03.87

(51) Int. Cl.$^3$: A 61 K 31/71

(30) Priority: 03.04.86 US 847787

(43) Date of publication of application: 07.10.87 Bulletin 87/41

(84) Designated Contracting States: BE DE FR GB NL

(71) Applicant: MERCK & CO. INC.
126, East Lincoln Avenue P.O. Box 2000
Rahway New Jersey 07065-0900(US)

(72) Inventor: Dinetta, Joseph
380 Johnston Drive
Watchung, New Jersey 07060(US)

(72) Inventor: Lankas, George R.
15 Hidden Valley Drive
Solebury, Pennsylvania 18913(US)

(74) Representative: Hesketh, Alan, Dr.
European Patent Department Merck & Co., Inc. Terlings Park Eastwick Road
Harlow Essex, CM20 2QR(GB)

(54) **Avermectins as growth promotant agents.**

(57) There is disclosed the effect that in otherwise healthy animals, the administration of avermectin compounds has the effect of increasing the growth and feed utilization of such animals beyond that which would be expected from nontreated animals.

EP 0 240 274 A2

2259S/1112A



TITLE OF THE INVENTION

AVERMECTINS AS GROWTH PROMOTANT AGENTS

BACKGROUND OF THE INVENTION

Avermectin and milbemycin compounds have been known for some time as having superior anthelmintic and antiparasitic effects. See U.S. Patents 4,310,519 to Albers-Schonberg *et al*., 4,199,569 to Chabala *et al*. and 3,950,360 to Aoki *et al*. The use of such compounds in parasitised animals generally results in an increase in weight which has been attributed to the general improvement of the health of the animal as the parasite burden is reduced. Now it has been observed that the administration of avermectin or milbemycin compounds to parasite-free animals results in a significant weight gain which can be only attributed to the use of the avermectin or milbemycin itself.

SUMMARY OF THE INVENTION

It has been observed that avermectin and milbemycin compounds have significant growth promoting effects when administered to parasite-free animals or to animals without clinical signs of parasitic infection. Thus, in addition to their known antiparasitic effects, such compounds are proposed as being growth promotant agents. Thus, it is an object of this invention to describe the growth promotant effects of avermectin and milbemycin compounds. A further object is to describe compositions and formulations for improved growth promotion using avermectin and milbemycin compounds as the active ingredient. Further objects will become apparent from a reading of the following description.

DESCRIPTION OF THE INVENTION

Avermectin compounds have been discovered to have significant growth promotant effects in animals. The avermectin compounds are a series of compounds derived from the original avermectin natural products isolated from a fermentation broth and described in U.S. Patent 4,310,519 to Albers-Schonberg *et al*. The avermectins are isolated as four pairs of compounds and the pair identified as Bla/Blb is the most preferred. The 22,23-dihydro derivatives of the avermectins are disclosed in 4,199,569 to Chabala *et al*. and the 22,23-dihydro avermectin Bla/Blb pair of compounds in an approximate 80:20 mixture are preferred and are known as ivermectin.

Other avermectin derivatives are useful as growth promotant agents such as monosaccharide and aglycone derivatives disclosed in U.S. Patent 4,206,205 to Mrozik et al; the acylated derivatives thereof such as those disclosed in U.S. Patent 4,201,861 to Mrozik et al; the 13-deoxy aglycone compounds disclosed in Re 32034 and Re 32006; and the 4"-keto and 4"-amino compounds disclosed in U.S. Patent 4,427,663 to Mrozik.

Additional compounds usable as growth promotant agents are the milbemycin compounds disclosed in U.S. Patent 3,950,360 to Aoki et al. and the oxime derivatives thereof disclosed in U.S. Patent 4,547,520 to Ide et al.

The preferred avermectin compounds for use as growth promotant agents are realized in the following structural formula:

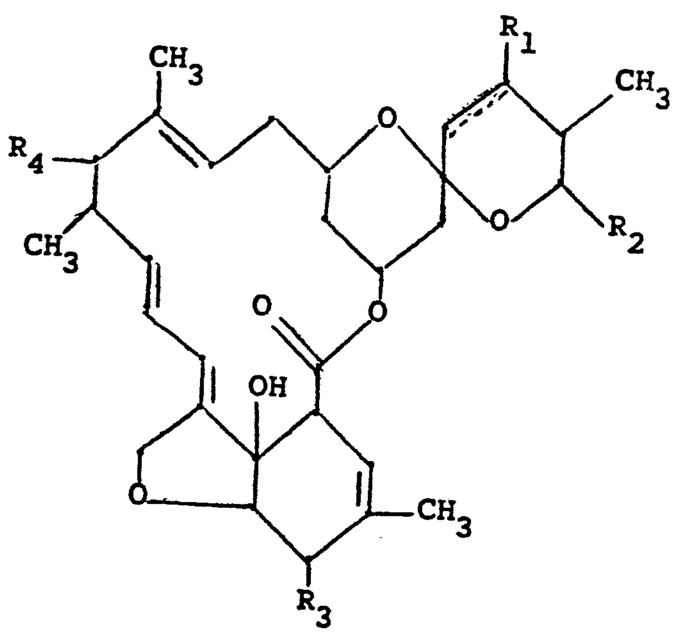

wherein the broken line indicates a single or double bond;

$R_1$ is H, =O, loweralkanoyloxy or OH, provided that $R_1$ is present only when the broken line indicates a single bond;

$R_2$ is methyl, ethyl, isopropyl or sec-butyl;

$R_3$ is OH, $OCH_3$ or loweralkanoyloxy;

$R_4$ is H, OH, loweralkanoyloxy, α-L-oleandrosyloxy, 4'-(α-L-oleandrosyl)-α-L-oleandrosyloxy, 4'-loweralkanoyl-α-L-oleandrosyloxy, 4"-loweralkanoyl-4'-(α-L-oleandrosyl)-α-L-oleandrosyloxy, 4"-amino-4'-(α-L-oleandrosyl)-α-L-oleandrosyloxy, 4"-mono- or diloweralkylamino-4'-(α-L-oleandrosyl)-α-L-oleandrosyloxy,

and physiologically acceptable salts thereof.

The preferred milbemycin compounds for use as growth promotion agents are realized in the following formula:

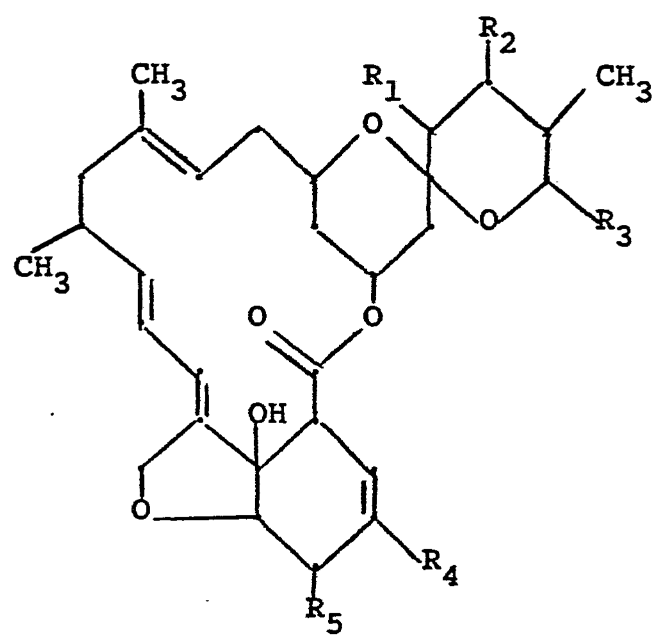

wherein the various R groups have the following meanings:

| $R_1$ | $R_2$ | $R_3$ | $R_4$ | $R_5$ |
|---|---|---|---|---|
| H | H | $CH_3$ | $CH_3$ | OH |
| H | H | $CH_3$ | $CH_3$ | $OCH_3$ |
| H | H | $C_2H_5$ | $CH_3$ | OH |
| H | H | $C_2H_5$ | $CH_3$ | $OCH_3$ |
| OH | $-O-\overset{O}{\overset{\Vert}{C}}-\overset{CH_3}{CH}-C_4H_9$ | $CH_3$ | $CH_3$ | OH |
| OH | $-O-\overset{O}{\overset{\Vert}{C}}-\overset{CH_3}{CH}-C_4H_9$ | $CH_3$ | $CH_3$ | $OCH_3$ |
| OH | $-O-\overset{O}{\overset{\Vert}{C}}-\overset{CH_3}{CH}-C_4H_9$ | $C_2H_5$ | $CH_3$ | OH |
| OH | $-O-\overset{O}{\overset{\Vert}{C}}-\overset{CH_3}{CH}-C_4H_9$ | $C_2H_5$ | $CH_3$ | $OCH_3$ |
| H | H | $CH_3$ | 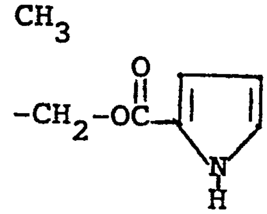 | OH |
| H | H | $C_2H_5$ | 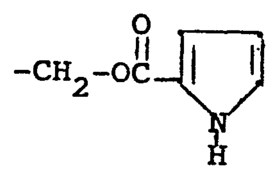 | OH |
| H | H | $i-C_3H_7$ | 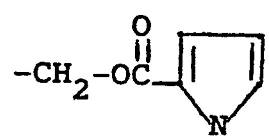 | OH |
| H | H | $CH_3$ | $CH_3$ | $=N-OR_6$ |
| H | H | $C_2H_5$ | $CH_3$ | $=N-OR_6$ |
| H | H | $i-C_3H_7$ | $CH_3$ | $=N-OR_6$ |

wherein $R_6$ is hydrogen or loweralkyl.

The term "loweralkyl" when used in the instant application is intended to represent those alkyl groups either straight or branched chain which have from 1-5 carbon atoms. Examples of such alkyl groups are methyl, ethyl, propyl, iso-propyl, butyl, sec-butyl, pentyl, and the like.

The term "loweralkanoyl" is intended to include those alkanoyl groups containing from one to five carbon atoms in either a straight or branched chain. Examples of such alkanoyl groups are formyl, acetyl, propionyl, butyryl, valeryl, and the like.

The "b" compounds, those with a 25-isopropyl group, are difficult to separate from the corresponding "a" compounds with a 25-sec-butyl group and as such the compounds are generally isolated as mixtures of the two compounds. Thus references in the instant application to "a" compounds such as B1a, A1a, and the like, are construed to define the pure compound as well as those which actually contain a certain proportion of the corresponding "b" compound. Alternatively, this representation of a mixture is sometimes done by referring to the B1 or B2 compounds or by separating the "a" compound from the "b" compound by a slash (/) such as B1a/B1b, B2a/B2b and the like.

The avermectin compounds can be used to increase the growth and feed efficiency of ruminant and non-ruminant animals such as sheep, cattle, goats, horses, swine, chickens and the like. The active compound can be fed to the animal by incorporating it into the animal's feed or drinking water or it can be administered in a unit dosage form

either orally as a drench, tablet, bolus or sustained release bolus or parenterally by injection or from a subcutaneous implant. The administration of the active compounds will produce a surprising increase in body weight, decrease in body fat and increase in body protein for the same food intake.

The active compounds can be administered to the animals at daily rates of from 0.02 to 2.0 mg/kg of body weight which may vary depending upon the particular animal being treated as well as the age and general physical condition of the animal. Preferably, daily dosages of from 0.1 to 1.0 mg/kg are utilized. When administered as part of the animal's feed or drinking water the active compound is present at rates of from 0.1 to 100 ppm which is determined to provide the appropriate daily amounts of the growth promotant compound.

The growth promotant effects of the instant compounds have been demonstrated in rats given 0, 0.8 and 1.6 mg/kg/day for a 14-week test period. At the end of the test period the treated animals were observed to have from 6 to 10% greater weight gain when compared with the control groups.

Similarly in a 60-week study dosages of 0.25, 1.5 and 2.0 mg/kg/day produced 30, 14 and 13% increased weight gain respectively when compared with control groups.

At the same dosages listed above for growth promotion effects, substantial increases in feed efficiency are also observed.

CLAIMS:

1. The use of an avermectin or a milbemycin compound for the manufacture of a medicament useful for increasing the growth and feed efficiency in animals.

2. The use as claimed in Claim 1 wherein the active compound has the formula:

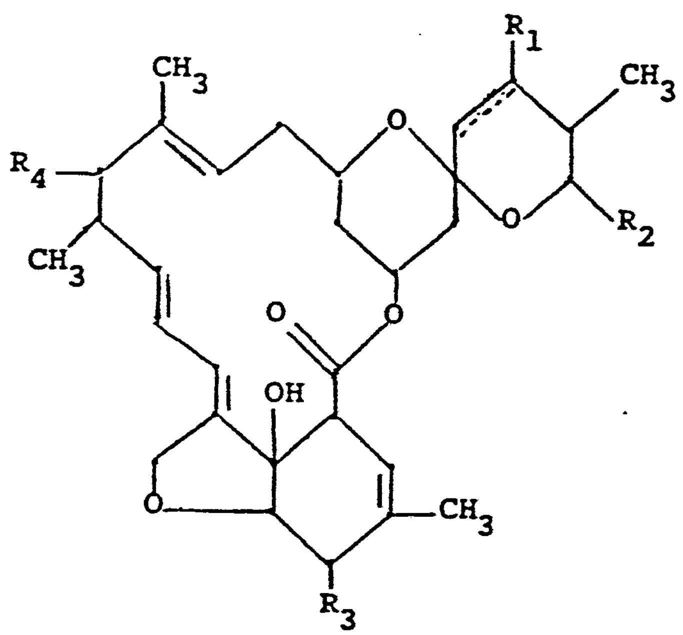

wherein the broken line indicates a single or double bond;

$R_1$ is H, =O, loweralkanoyloxy or OH, provided that $R_1$ is present only when the broken line indicates a single bond;

$R_2$ is methyl, ethyl, isopropyl or sec-butyl;

$R_3$ is OH, $OCH_3$ or loweralkanoyloxy;

$R_4$ is H, OH, loweralkanoyloxy, α-L-oleandrosyloxy, 4'-(α-L-oleandrosyl)-α-L-oleandrosyloxy, 4'-loweralkanoyl-α-L-oleandrosyloxy, 4"-loweralkanoyl-4'-(α-L-oleandrosyl)-α-L-oleandrosyloxy, 4"-amino-4'-(α-L-oleandrosyl)-α-L-oleandrosyloxy, 4"-mono- or diloweralkylamino-4'-(α-L-oleandrosyl)-α-L-oleandrosyloxy,

and physiologically acceptable salts thereof.

3. The use as claimed in Claim 2 wherein the active compound is ivermectin.

4. The use as claimed in Claim 1 wherein the active compound has the formula:

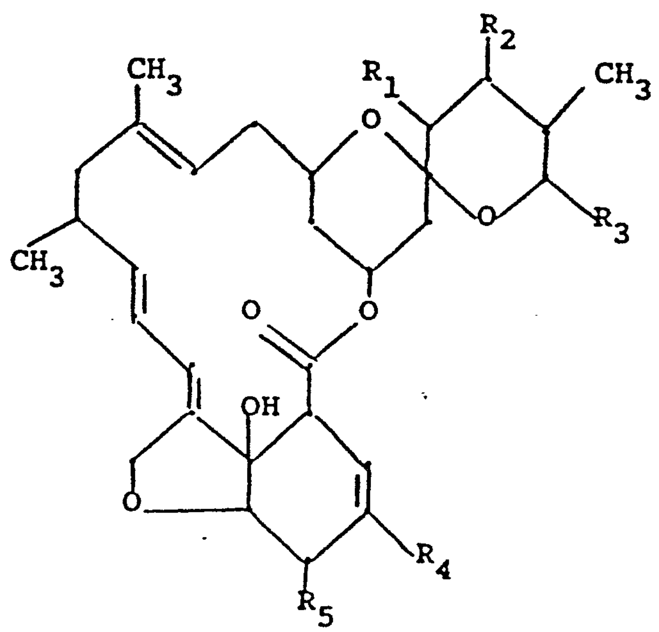

wherein the various R groups have the following meanings:

| $R_1$ | $R_2$ | $R_3$ | $R_4$ | $R_5$ |
|---|---|---|---|---|
| H | H | $CH_3$ | $CH_3$ | OH |
| H | H | $CH_3$ | $CH_3$ | $OCH_3$ |
| H | H | $C_2H_5$ | $CH_3$ | OH |
| H | H | $C_2H_5$ | $CH_3$ | $OCH_3$ |
| OH | 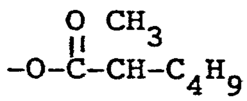 | $CH_3$ | $CH_3$ | OH |
| OH | 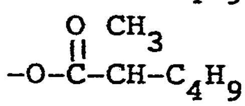 | $CH_3$ | $CH_3$ | $OCH_3$ |
| OH | 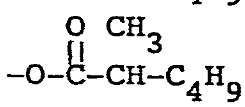 | $C_2H_5$ | $CH_3$ | OH |
| OH | 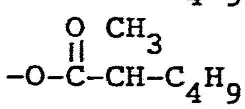 | $C_2H_5$ | $CH_3$ | $OCH_3$ |
| H | H | $CH_3$ | 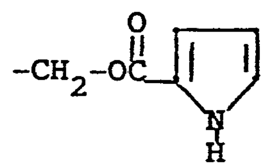 | OH |
| H | H | $C_2H_5$ | 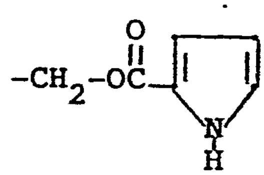 | OH |
| H | H | $i-C_3H_7$ | 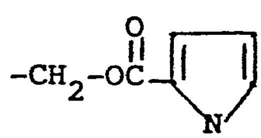 | OH |
| H | H | $CH_3$ | $CH_3$ | $=N-OR_6$ |
| H | H | $C_2H_5$ | $CH_3$ | $=N-OR_6$ |
| H | H | $i-C_3H_7$ | $CH_3$ | $=N-OR_6$ |

wherein $R_6$ is hydrogen or loweralkyl.

5. The use as claimed in any one of Claims 1 to 4 wherein the medicament is suitable for administering the active compound at a dosage of from 0.1 to 1.0 mg/kg/day of the animal's body weight.

6. The use as claimed in any one of Claims 1 to 5 wherein the medicament is in a unit dosage form selected from a drench, tablet, bolus or sustained release bolus.

7. The use as claimed in any one of Claims 1 to 5 wherein the medicament is a subcutaneous implant.

8. A composition useful for increasing the growth and feed efficiency of animals which comprises an inert ingredient and an avermectin or milbemycin compound.

9. The composition of Claim 8 which is a medicated feed or drinking water.

10. The composition of Claim 8 which is a unit dosage formulation selected from a drench, tablet, bolus or sustained release bolus.